# EUROPEAN PATENT SPECIFICATION

(11) **EP 4 370 593 B1**
(45) Date of publication and mention of the grant of the patent: **10.09.2025**
(21) Application number: 22750831.4
(22) Date of filing: 15.07.2022
(51) Int. Cl.: C08J 11/10, C12P 7/00, B09B 3/00, C08J 7/12

(54) **METHOD TO DEGRADE PLASTIC**
VERFAHREN ZUM ABBAU VON KUNSTSTOFF
PROCÉDÉ DE DÉGRADATION DU PLASTIQUE

(30) Priority: 16.07.2021 EP 21382643
(43) Date of publication of application: 22.05.2024
(73) Proprietor: Repsol, S.A., 28045 Madrid (ES); Evoenzyme, S.L., 28341 Madrid (ES)
(72) Inventor: GONZÁLEZ BARROSO, Maria del Mar, 28935 MÓSTOLES (ES); TORRES SALAS, Pamela, 28935 MÓSTOLES (ES); ESPI GUZMAN, Enrique, 28935 MÓSTOLES (ES); GÓMEZ FERNÁNDEZ, Bernardo José, 28049 MADRID (ES); VIÑA GONZÁLEZ, Javier, 28049 MADRID (ES); MATELJAK, Ivan, 28049 MADRID (ES)
(74) Representative: ZBM Patents - Zea, Barlocci & Markvardsen
(86) International application number: PCT/EP2022/069842
(87) International publication number: WO 2023/285648

(56) References cited:
- WO-A1-2008/012236
- US-A1- 2020 406 320
- SAKHALKAR SACHIN ET AL: "Screening and Identification of Soil Fungi with Potential of Plastic Degrading Ability", INDIAN JOURNAL OF APPLIED RESEARCH, 31 December 2013 (2013-12-31), pages 62 - 64, XP093005905
- SCHEIBNER KATRIN ET AL: "Unspezifische Peroxygenasen - Oxyfunktionalisierung außerhalb der Pilzhyphe", BIOSPEKTRUM, SPEKTRUM AKADEMISCHER VERLAG, DE, vol. 26, no. 1, 1 February 2020 (2020-02-01), pages 103 - 106, XP037022720, ISSN: 0947-0867, [retrieved on 20200215], DOI: 10.1007/S12268-020-1338-X

## Description

### Technical Field

The present application refers to the field of plastic degradation. In particular, it refers to a method for degrading polyolefins, such as polyethylene, by using unspecific peroxygenases.

### Background Art

Plastics are ubiquitous in our society. They are the basic material for innumerable commodity products. Plastics are inexpensive, each is a highly engineered material with precise physical properties. They can be molded into virtually any desired shape through rotation, injection, extrusion, compression, blowing, or thermoforming. Their material properties are adjusted during and/or after synthesis to achieve the desired strength, permeability, porosity, opacity, and color.

Polyolefin plastics are particularly durable, due to their chemical and biological inertness, which is a result of their high molecular weight and hydrophobicity, and the absence of functional groups that are susceptible to attack by microbial enzymes, light, water, etc. The recalcitrance and impermeability of these plastics make them ideal for applications such as food packaging, sterile medical uses, and construction, among others, but also make them particularly long-lived when they are discarded. Various antioxidants and stabilizers, which are used to prolong the working life of plastics, slow environmental degradation of plastics waste even further.

Consequently, the very properties that make polyolefin plastics so versatile for humans has also created an emerging threat to the environment. As one of the most recalcitrant plastics, polyolefin plastics and, in particular, polyethylene, make up a substantial portion of the world's plastic waste.

Although a small portion of the plastic waste is recycled or disposed of by combustion, most of the waste is buried in landfills. Burning plastic waste is expensive and releases toxic gases, including dioxins and furans. Most plastic waste ends up in landfill sites or in the ocean, which leads to serious environmental problems.

Generally speaking, natural degradation of plastic begins with photodegradation, which leads to thermooxidative degradation. Ultraviolet light from the sun provides the activation energy required to initiate the incorporation of oxygen atoms into the polymer. This causes the plastic to become brittle and to break into smaller and smaller pieces. At the same time, environmental microorganisms colonize the plastic surface and either convert the carbon in the polymer chains to carbon dioxide or incorporate it into biomolecules.

However, this entire process is very slow, particularly in the case of polyolefins, for which it can take 50 or more years. This is not aided by the fact that the photodegradative effect is significantly decreased when the plastic becomes buried. The situation is even worst for plastic waste dumped in seawater due to the lower temperature and oxygen availability. The rate of hydrolysis of most polymers is insignificant in the ocean, which does not help degradation.

There is an urgent need to develop efficient methods to accelerate plastic degradation and, in particular, to degrade polyolefins, such as polyethylene.

### Summary of Invention

The inventors have surprisingly found that short unspecific peroxygenases (UPOs) catalyze significant alterations of the polyolefin structure. In particular, short UPOs catalyze oxyfunctionalization of the polyolefin structure, introducing oxygenated functional groups that render the polyolefin more amenable to further modifications. As shown in the examples below, this particular type of enzyme is able to alter the chemical structure of a highly recalcitrant plastic, namely, polyethylene. As a result, the degradation (or other further processing) of recalcitrant polyolefins, benefits from treatment of the polyolefin with a short unspecific peroxygenase (short UPO) enzyme.

Thus, a first aspect of the invention provides method for oxyfunctionalization of polyolefin, said method comprising the step of (i) contacting the polyolefin with an isolated short unspecific peroxygenase enzyme (short UPO) in aqueous media and in the presence of an hydroperoxide , wherein the concentration of hydroperoxide ranges from 0.1 to 10 mM.

This first method reduces hydrophobicity of a polyolefin.

The effect of the short UPO on the plastic may be measured by Fourier-transform infrared spectroscopy (FTIR) and, in particular, by attenuated total reflection-FTIR (ATR_FTIR). The examples below show the alterations on polyethylene structure after treatment with a short UPO as measured by FTIR. The results show that treatment with short UPO results in the emergence of a large signal in the 1705-1740 cm-1 region of the spectrum (corresponding to carbonyl group) and several other signals in the region between 1400-900 cm-1. Such a high degree of oxyfunctionalization is not found with related enzymes, such as long UPO or laccase. The high alteration in the 1400-900 cm-1 region is specific for treatment with short UPO. Moreover, the shape and location of the carbonyl peak is also very specific for treatment with short UPO. The carbonyl peak appears specifically in the region going from 1724 to 1740 cm-1, while its shape may indicate that there is more than one carbonyl group, which is also quite specific for short UPO enzymatic treatment. This degree of functionalization indicates that a high diversity of oxygen-containing functional groups may be introduced by short UPO in the polymer structure, increasing the amenability of the treated polyolefin to further modifications and consequently accelerating degradation or further processing.

A second aspect of the invention provides a method for degrading polyolefin, said method comprising the step of (i) contacting the polyolefin with an isolated short unspecific peroxygenase enzyme (short UPO) in aqueous media and in the presence of an hydroperoxide, wherein the concentration of hydroperoxide ranges from 0.1 to 10 mM. That is, the second aspect provides a method for degrading polyolefin comprising oxyfunctionalization of the plastic as defined in the first aspect.

As mentioned above, the contacting of the short UPO with the plastic substantially accelerates the degradation of said polyolefin. Thus this second aspect could also be envisioned as a method to accelerate polyolefin degradation, said method comprising the step of (i) contacting the polyolefin with an isolated short unspecific peroxygenase enzyme (short UPO) in the presence of an hydroperoxide , wherein the concentration of hydroperoxide ranges from 0.1 to 10 mM.

The alterations enabled by the short UPO in the polyolefin structure are further useful for a range of applications. Notably, treatment with short UPO is useful in polyolefin revalorization processes by which polyolefin is generally depolymerized to yield useful intermediate products of lower molecular weight, such as dicarboxylic acids, or products that are useful as feedstocks for microbial production of chemicals with high value. Also, biological recycling of polyolefin may also benefit from the oxyfunctionalization achieved by treatment with a short UPO.

Thus, a third aspect of the invention refers to a method for degrading or depolymerizing a polyolefin, said method comprising oxyfunctionalization of the polyolefin as defined in the first aspect of the invention.

The polyolefin obtained by the method of the first aspect is an oxyfunctionalized polyolefin, i.e. a plastic that contains a higher degree of oxygen-containing functional groups as compared to the substrate polyolefin.

In a fourth aspect, the present invention discloses the use of an isolated short UPO in polyolefin oxyfunctionalization, in polyolefin degradation, in polyolefin depolymerization, wherein the use is in aqueous media and in the presence of an hydroperoxide.

### Brief Description of Drawings

FIG 1. Conserved amino acid residues in the active site of short UPO from *Marasmius rotula* (MroUPO, UPO3).
FIG 2. Enzymatic modification of LDPE by short UPO from *Marasmius rotula* (UPO3) vs Control.
FIG 3. Enzymatic modification of LDPE by short UPO from *Marasmius rotula* (UPO3) vs long UPO from *Agrocybe aegerita* (UPO1).
FIG 4. Enzymatic modification of LDPE by short UPO from *Marasmius rotula* (UPO3) vs Laccase. A, region 2000-800 cm-1. B, region 1400-900 cm-1.
FIG 5. Enzymatic modification of LDPE by short UPO from *Marasmius rotula* (UPO3) vs Versatile Peroxidase
FIG 6. Enzymatic modification of LDPE by short UPO from *Marasmius rotula* (UPO3) vs thermo-oxidation.

### Detailed description of the invention

All terms as used in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

As mentioned above, the invention is based in use of a short unspecific peroxygenase for degrading plastic, or for accelerating plastic degradation. It is hypothesized that the degree and variety of alterations in the plastic structure effected by the activity of the short UPO result in enhanced degradation of the plastic.

The term "oxyfunctionalization" refers to the insertion of oxygen-containing functional groups in a substrate chemical molecule. This is particularly difficult to achieve in highly recalcitrant and hydrophobic substrates such as polyolefins. Non-limiting oxygen-containing functional groups that may be introduced to the plastic, and in particular, to polyolefin plastic, according to the method of the invention are hydroxyl (ROH), carbonyl (RCOR') (aldehyde (RCHO), carboxyl (RCOOH), carboxylate (RCOO⁻), ester (RCOOR'), peroxy (ROOR'), hydroperoxyl (ROOH), carbonate ester (ROCOOR'), ether (ROR'), acetal (RCH(OR')(OR")), hemiacetal (R2CH(OR1)(OH)), and epoxide. In particular, oxyfunctionalization in the sense of the present invention comprises the insertion of carbonyl groups in a substrate polyolefin, and more particularly, comprises the insertion of ester groups.

Unspecific peroxygenases (UPO, EC.1.11.2.1), also known as aromatic peroxygenases (APO), are newly discovered extracellular enzymes which belong to heme-thiolate proteins obtained from fungal species. Peroxygenases catalyze the insertion of an oxygen atom from H₂O₂ or other organic peroxide which acts as a source of oxygen, or O₂ itself, in a wide variety of substrates. Based on their molecular mass and motif patterns, UPOs are classified into two categories: Group-I (short UPO sequences) with an average mass of 29 kDa and Group-II (long UPO sequences) with an average mass of 44 kDa (Hofrichter et al., 2015). The latter are almost exclusively found in ascomycetes and basidiomycetes, while the former are distributed among all fungal phyla. Characterized UPO from *Marasmius rotula* (MroUPO) belongs to the short UPO group, while the UPO from *Agrocybe aegerita* (AaeUPO) belongs to the long UPOs. It is to be noted that the size of the UPOs as referred above corresponds to monomeric molecular weight. For example, short UPOs are generally formed by two monomers (homodimer), with molecular weights of ~29 kDa per monomer.

Importantly, differences between short and long UPOs exist also in the catalytic sites. The heme access channel is where the majority of the differences between short and long UPOs arise. In short UPOs, the hydrophobic amino acids that conform the active site are aliphatic making a wider, yet shorter access channel when compared with long UPOs (whose active site is formed by aromatic residues). This disposition of the active site in short UPOs explains their strong preference for bulky substrates. Short UPOs have a conserved motif in the catalytic site: -PCP- and -EHD-S-[E], a glutamic acid acting as the acid-base catalyst in the cleavage of H₂O₂ and histidine as charge stabilizer. Said motif is not found in long UPOs. For example, long UPOs of the AaeUPO contain the consensus - PCP-and EGD-R-E-.

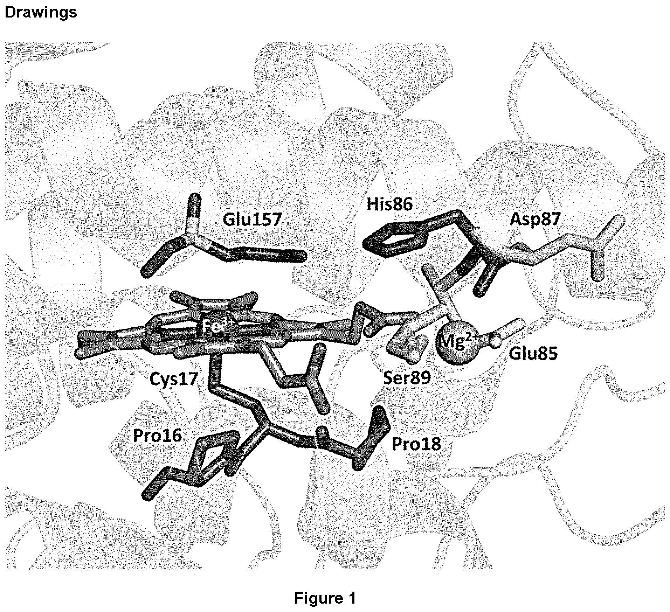

In the active site is also located the heme group (iron protoporphyrin IX), coordinated through a cysteine as proximal (5th) ligand (heme-thiolate) that with two proline residues form the PCP motif, which places the thiolate (Cys-SH) towards the heme iron. When the enzyme is in the resting state, there is a water molecule (6th) as ligand in the distal heme position. This molecule of water is substituted by hydrogen peroxide (electron accepting co-substrate) at the beginning of the catalytic cycle. In the surrounding area of the heme group, there is a structural magnesium ion (Mg2+) coordinated through three carboxylates and an alcohol group from heme propionate -glutamate, aspartate, and serine. The main role of the magnesium ion is to stabilize the porphyrin system (figure 1).

The activity of short UPOs may be assayed, for example, using colorimetric substrates, such as 2,6-dimethoxyphenol (see examples below for assay conditions). However, these colorimetric substrates may also be used to measure other oxidoreductase activities. A more specific assay to determine short UPO activity is the cleavage reaction of model corticosteroids described in Ullrich et al, 2018 (J. Inorg. Biochem. 183, 84-93).

In the present invention isolated short UPO are used.

Short UPOs have been reported from various organisms, mostly from fungus (Hofrichter et al., 2015, *supra*)*.* Thus, in some embodiments of any aspect of the present invention, optionally in combination with any one of the embodiments provided below, the short UPO is from a fungus. Non-limiting fungal short UPOs include those from Basidiomycota, Ascomycota, Oomycota, Zygomycota, Chytridiomycota, and Glomeromycota. In particular embodiments of any aspect of the invention, optionally in combination with any one of the embodiments provided below, the short UPO is selected from *Marasmius rotula, Marasmius wettsteinii, Chaetomium globosum, Daldinia caldariorum* and *Collariella virescens.* and others disclosed in Hofrichter et al., 2015 (*supra*)*.* In more particular embodiments, the short UPO is from a Basidiomycota fungus, for example, from *Marasmius rotula or Marasmius wettsteinii.*

In some embodiments of any aspect of the present invention, optionally in combination with any one of the embodiments provided below, the short UPO has a monomer size around 29 kDa, for example, ranging from 25 to 35 kDa, or from 27 to 33 kDa, for example, 28 kDa, 29 kDa, 30 kDa, 31 kDa, or 32 kDa. In particular embodiments, optionally in combination with any one of the embodiments provided below, the short UPO is a polypeptide showing unspecific peroxygenase activity having a monomer size between 25 to 35 kDa, or from 27 to 33 kDa, for example, 28 kDa, 29 kDa, 30 kDa, 31 kDa, or 32 kDa.

The catalytic site of the short UPO comprises a) the conserved motif PCP and -EHD-S-[E]-, and b) glutamic acid and histidine as acid-base pair. The skilled person would expect any enzyme containing the same catalytic site to have the same activity. Thus enzymes containing the short UPO conserved motif in the catalytic site are expected to have essentially the same oxyfunctionalization activity as, for example, *Marasmius rotula.*

A sequence motif is an amino-acid sequence pattern that is widespread and usually assumed to be related to biological function of the macromolecule. A conserved sequence motif, or simply, conserved motif, is a sequence motif that is identical, or highly similar, among proteins of different species, or within a genome. The catalytic site conserved motif mentioned above for short UPOs is the conserved motive shared among substantially all short UPOs from different organisms, particularly fungus.

In other embodiments of any aspect of the present invention, optionally in combination with any one of the embodiments provided above or below, the short UPO has a sequence similarity of at least 60%, with SEQ ID NO: 1. In particular, the short UPO has a sequence similarity of at least 70% with SEQ ID NO: 1. More particularly, the short UPO has a sequence similarity of at least 75%, 80%, 81%, 82%, 83%, 84%, 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5% with SEQ ID NO: 1.

In other embodiments of any aspect of the present invention, optionally in combination with any one of the embodiments provided above or below, the short UPO has a sequence identity of at least 40% with SEQ ID NO: 1. In particular, the short UPO has a sequence identity of at least 60% with SEQ ID NO: 1. More particularly, the short UPO has a sequence identity of at least 70% with SEQ ID NO: 1. Even more particularly, the short UPO has a sequence identity of at least 75%, 80%, 81%, 82%, 83%, 84%, 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5%, or 100% with SEQ ID NO: 1.

In the present invention the term "identity" refers to the percentage of residues that are identical in the two sequences when the sequences are optimally aligned. The percentage of identity determines the number of identical residues over a defined length in a given alignment. Thus, the level of identity between two sequences or ("percent sequence identity") is measured as a ratio of the number of identical positions shared by the sequences with respect to the number of positions compared (i.e., percent sequence identity = (number of identical positions/total number of positions compared) x 100). A gap, i.e., a position in an alignment where a residue is present in one sequence but not in the other, is regarded as a position with non-identical residues and is counted as a compared position.

A number of mathematical algorithms for rapidly obtaining the optimal alignment and calculating identity between two or more sequences are known and incorporated into a number of available software programs. For example, the sequence identity between two amino acid sequences may be determined using algorithms based on global alignment, such as the Needleman-Wunsch algorithm (Needleman and Wunsch, 1970, J. Mol. Biol. 48: 443-453), such as those implemented in the Needle program of the EMBOSS package (EMBOSS: The European Molecular Biology Open Software Suite, Rice et al., 2000, Trends Genet. 16: 276-277); or the BLAST Global Alignment tool (Altschul et al., "Basic local alignment search tool", 1990, J. Mol. Biol, v. 215, pages 403-410), using default settings. Local alignment also can be used when the sequences being compared are substantially the same length.

The term similarity allows for conservative substitutions of amino acid residues having similar physicochemical properties over a defined length of a given alignment.

According to the present invention, the plastic is a polyolefin, for example, selected from the group consisting of polyethylene, polypropylene, polymethylpentene, polybutene-1, ethylene-octene copolymers, stereo-block polypropylene (PP), olefin block copolymers, propylene-butane copolymers, polyolefin elastomers, polyisobutylene, poly(alpha-olefin)s, ethylene propylene rubber, ethylene propylene diene monomer (M-class) rubber, and any combination thereof. In particular embodiments of any aspect of the present invention, optionally in combination with any one of the embodiments provided above or below, the plastic is polyethylene, for example selected from the group consisting of linear low density polyethylene (LLDPE), high density polyethylene (HDPE), low density polyethylene (LDPE), ultra high molecular weight polyethylene (UHMWPE), medium density polyethylene (MDPE), ethylene vinyl acetate (EVA), ethylene butyl acrylate (EBA), and any combination thereof.

In some embodiments of any aspect of the present invention, optionally in combination with any one of the embodiments provided above or below, the plastic may take any form such as emulsion, solid pellet or a film.

A first aspect of the invention is directed to a method for oxyfunctionalization of plastic, said method comprising the step of (i) contacting the plastic with an isolated short unspecific peroxygenase enzyme (short UPO) in aqueous media and in the presence of an hydroperoxide, wherein the concentration of hydroperoxide ranges from 0.1 to 10 mM, in particular from 1 to 10 mM.

The conditions for the contacting step in the first aspect of the invention are optimized for short UPO activity.

Presence of an oxygen donor is required for peroxygenase activity. The term "oxygen donor", "oxidising agent" and "oxidant" relate to a substance, molecule or compound that donates oxygen to a substrate in an oxidation reaction. Typically, the oxygen donor is reduced (it accepts electrons). By way of example, non-limiting oxygen donors include molecular oxygen or dioxygen (O₂) and peroxides. A "peroxide" is any compound other than molecular oxygen (O₂) which has two oxygen atoms bonded to each other (ROOR').

The method of the first aspect of the invention thus requires presence of an oxygen donor which is a hydroperoxide. The invention also contemplates combination of a hydroperoxide and O₂.

The concentration of hydroperoxide ranges from 0.1 to 10 mM. In particular, the concentration of peroxide ranges from 0.5 to 10 mM, or from 1 to 10 mM, or from 2 to 9 mM, or from 3 to 8 mM, for example 4 mM, 5 mM, 6 mM, or 7 mM. The hydroperoxide may be provided as direct reagent in the contacting step or may be indirectly provided, for example by combining UPOs with photo-, electro- and chemo-catalysis, as well as by using enzyme cascade reactions all of them aimed at controlling the gradual supply of H₂O₂ in situ (Hobisch et al., 2020; van Schie et al., 2020).

A peroxide is generally represented by formula ROOR', where R and R' are independently selected from H, (C₁-C₁₀)-alkyl or, (C₁-C₁₀)-alcoxy, and (C₁-C₁₀)-aryl. The expression "(Cₓ-C_{y})-alkyl" as used herein refers to a saturated branched, cyclic or linear hydrocarbon side chain with x to y carbon atoms. For example, "(Cₓ-C_{y})-alkyl" may be (C₁-C₄)-alkyl, which is preferably an unsubstituted group selected from methyl, ethyl, propyl, isopropyl, butyl, isobutyl, s-butyl, and t-butyl. The term "aryl" refers to a radical of one ring system with 1-3 rings, the rings being aromatic and being isolated or partially/totally fused and having 5-6 members, being each member independently selected from C, CH, N, NH, O, S where chemically possible, and the ring system being optionally substituted by one or more radicals independently selected from the group consisting of (C₁-C₆)alkyl, (C₁-C₆)alkoxy, nitro, cyano, and halogen. R and R' can be independently selected from H, (C₁-C₁₀)-alkyl, and (C₁-C₁₀)-aryl, wherein alkyl is linear or branched and aryl is phenyl or (C₁-C₆)-phenyl. In the present invention a hydroxyperoxyde is used. A hydroperoxide has general formula R-O-O-R', wherein at least R or R' are H (also called peroxol). In more particular embodiments, the peroxide is selected from the group consisting of H₂O₂, tert butyl hydroperoxide, paracetic acid, and cumene hydroperoxide. In more particular embodiments, the peroxide is hydrogen peroxide or tert butyl hydroperoxide, more preferably hydrogen peroxide.

The method of the first aspect of the invention comprises aqueous media. By "aqueous media" it is understood a medium that comprises water. In some embodiments of the first aspect, optionally in combination with any one of the embodiments provided above or below, the medium comprises at least 10%, or at least 20% or at least 30%, or at least 40%, or at least 50%, or at least 60%, or at least 70%, or at least 80% or at least 90%, or 100% water. In some embodiments, optionally in combination with any one of the embodiments provided above or below, the medium may comprise an organic phase, for example, acetonitrile, methanol, ethanol or acetone. Said organic phase may be in a concentration ranging from 0,5 to 50%, or from 0,5 to 20%, or from 0,5 to 10%, for example 1%, 2%, 3%, 4%, 5%, 6%, 7%, 8%, or 9%.

In another embodiment of the first aspect, optionally in combination with any one of the embodiments provided above or below, the pH at which the contacting step takes place ranges from 2 to 11, or from 2 to 9, or from 2 to 8, or from 2 to 7, or from 3 to 7. In particular, the pH ranges from 4 to 6, which is the best pH range for the activity of the short UPO enzyme, for example the pH may be 4.3, 4.5, 4.8, 5, 5.2, 5.5, or 5.7.

In order to maintain a convenient pH, the method of the invention may take place in the presence of a buffer. In some embodiments of the first aspect, optionally in combination with any one of the embodiments provided above or below, the contacting step takes place in the presence of a buffer. In particular, the buffer is one that provides a pH ranging from 2 to 9, or from 2 to 8, or from 2 to 7 or from 3 to 7, or from 4 to 6, for example a buffer that maintains a pH of 4.3, 4.5, 4.8, 5, 5.2, 5.5, or 5.7. The buffer may be selected from the non-limiting group consisting of citric acid, phosphate, maleate, and combinations thereof. In a particular embodiment of the first aspect of the invention, optionally in combination with any one of the embodiments provided above or below, the contacting step is done in the presence of citrate. In another particular embodiment, the contacting step is done in the presence of phosphate/citrate buffer.

In some embodiments of the first aspect, optionally in combination with any one of the embodiments provided above or below, the temperature at which the contacting step takes place ranges from 10 to 70 °C, in particular from 20 to 50 °C, or from 20 to 40 °C, for example, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 37 °C, 40°C, 41 °C, 42 °C, 43 °C, 44 °C, 45 °C, 46 °C, 47 °C, 48 °C, or 49 °C. In particular embodiments the temperature is room temperature.

Determination of an effective amount of short UPO for achieving the desired oxyfunctionalization in plastic is within the knowledge of one skilled in the art. Higher amounts of short UPO used in the contacting step will achieve a higher degree of alteration in a given reaction time. In some embodiments of the first aspect, optionally in combination with any one of the embodiments provided above or below, the amount of short UPO in the contacting step may range from 0.01 to 100 µM, or from 0.1 to 50 µM, or from 0.5 to 25 µM, or from 0,5 to 15 µM, or from 1 to 10 µM, for example 2 µM, 3 µM, 4 µM, 5 µM, 6 µM, 7 µM, or 8 µM.

The time required to produce a desirable degree of functionalization may depend, as will be apparent to the skilled person, on the amount of short UPO used in the method, as well as the reaction time and the temperature. In some embodiments of the first aspect, optionally in combination with any one of the embodiments provided above or below, the contacting step takes place for a time ranging from 12 hours to three months, or from one day to three months, or from two days to two months, or from three days to one month, or from three days to three weeks, or from five days to three weeks, or from one week to two weeks.

Other reagents may be added to the method of the first aspect of the invention in order to optimize the functionalization of the plastic.

It is also relevant that the carbonyl peak appears in the region from 1724 to 1740 cm-1 The oxyfunctionalization of the polyolefin may comprise the emergence of at least one peak in the region from 1705 to 1740 cm-1 in a ATR_FTIR spectrum and more than one peak in the region from 1400 to 900 cm-1 in a ATR_FTIR. In a particular embodiment of the first aspect of the invention, optionally in combination with any one of the embodiments provided above or below, the oxyfunctionalization comprises the emergence of at least one peak in the region from 1724 to 1740 cm-1 and more than one peak in the region from 1400 to 900 cm-1 in a ATR_FTIR. In particular embodiments of the first aspect of the invention, optionally in combination with any one of the embodiments provided above or below, the oxyfunctionalization comprises the emergence of at least one peak in the region from 1705 to 1740 cm-1 and at least two peaks, for example at least three, at least four, at least five, at least six, at least seven, at least eight, at least nine or at least ten peaks in the region from 1400 to 900 cm-1 in a ATR_FTIR. In particular embodiments of the first aspect of the invention, optionally in combination with any one of the embodiments provided above or below, the oxyfunctionalization comprises the emergence of at least one peak in the region from 1724 to 1740 cm-1 and at least two peaks, for example at least three, at least four, at least five, at least six, at least seven, at least eight, at least nine or at least ten peaks in the region from 1400 to 900 cm-1 in a ATR_FTIR. For example, the oxyfunctionalization comprises emergence of the following peaks in a ATR_FTIR spectrum: 1740 +/- 4 cm-1, 1724 +/- 4 cm-1, 1263.21 +/- 4 cm-1, 1229.50 +/- 4 cm-1, 1184.91 +/- 4 cm-1, 1133.02+/- 4 cm-1, 1100.82 +/- 4 cm-1, 1058.07 +/- 4 cm-1, 980.70 +/- 4 cm-1, and 894.71 +/- 4 cm-1. The oxyfunctionalization of the polyolefin as defined above results in a significant reduction of the recalcitrance of the polyolefin. In particular embodiments the substrate for the oxyfunctionalization as defined above is selected from polyethylene and polypropylene, in particular, polyethylene.

The method of the first aspect of the invention may also benefit from adding a further enzyme. Thus, in some embodiments of the first aspect of the invention, optionally in combination with any one of the embodiments provided above or below, the method further comprises a step (ii) of contacting the plastic with another enzyme. Appropriate enzymes to be used in the method of the invention are ligninolytic oxidoreductase enzymes different from short UPO, such as a long UPO, a laccase, a manganese peroxidase and a versatile peroxidase. Thus, in some embodiments of the first aspect of the invention, optionally in combination with any one of the embodiments provided above or below, the enzyme in step (ii) is a ligninolytic oxidoreductase enzyme different from short UPO, for example, a long UPO, a laccase, a manganese peroxidase and a versatile peroxidase. The step (ii) of contacting the plastic with another enzyme may take place before, after or at the same time as step (i).

The method according to the first aspect of the invention may comprise additional steps before and/or after step (i) of contacting the plastic with an isolated short UPO. For example, when the plastic to be treated is plastic waste, sorting of the plastic may be required. Moreover, the overall efficiency of the method of the invention may benefit from conventional pretreatment processes. Sometimes, decontamination of the plastic waste by conventional methods may be convenient prior to contacting with the short UPO. Thus, in some embodiments of the first aspect of the invention, optionally in combination with any one of the embodiments provided above or below, the method comprises at least one step prior to step (i), said prior step selected from the group consisting of sorting, decontaminating, milling, chemical hydrolysis, irradiation, heating and treating with pro-oxidants, for example, metal ions such as iron, manganese, titanium or cobalt.

A second aspect of the invention refers to a method for degrading polyolefin, said method comprising oxyfunctionalization of the polyolefin as defined in the first aspect. All embodiments defined for the first aspect are also applicable to the second aspect.

In some embodiments of the second aspect of the invention, optionally in combination with any one of the embodiments provided above or below, the method comprises at least further step, for example, conventional disposal of the enzyme-treated polyolefin. Disposal of the plastic usually comprises dumping to soil or landfills, where it comes into contact with environmental microorganisms.

In some embodiments of the second aspect of the invention, the method further comprises hydrolysis of the polyolefin structure. In other embodiments, the method further comprises disintegrating the plastic polyolefin into short chains of oligomers, dimers, and monomers. Said oligomers, dimers, and monomers may be subsequently used as source of carbon and energy to environmental microorganisms, for example, soil microorganisms. In another embodiment of the first aspect of the invention, the digested polyolefin is mineralized. In another embodiment of the second aspect of the invention, the method results in the end products carbon dioxide, water and/or methane.

The method of the second aspect of the invention is thus a method for the biodegradation of polyolefin, which is environmentally-friendly and economical.

The lower molecular weight oligomers or monomers that constitute the intermediate products of plastic biodegradation could also be exploited by themselves as relevant metabolites for industrial processes. This is the case of dicarboxylic acids, for example. The intermediate products of plastic depolymerization can also be used for the biosynthesis of high-value chemicals through specific metabolic pathways. This is known as a way of valorizing plastic wastes. In all cases, this method can benefit from treatment with short UPO. Accordingly, a third aspect of the invention refers to a method for depolymerization of plastic comprising oxyfunctionalization of the plastic by the method of the first aspect. All embodiments defined for the first aspect are also applicable to the third aspect.

The plastic obtained by the method of the first aspect is an oxyfunctionalized plastic, i.e. a plastic that contains a higher degree of oxygenated functional groups as compared to the substrate plastic.

Said plastic is polyolefin and has at least one peak in the region from 1705 to 1740 cm-1 in an ATR_FTIR spectrum and more than one peak in the region from 1400 to 900 cm-1 in an ATR_FTIR. In particular, the oxyfunctionalized polyolefin plastic has at least one peak in the region from 1724 to 1740 cm-1 and more than one peak in the region from 1400 to 900 cm-1 in a ATR_FTIR. In particular, the oxyfunctionalized polyolefin plastic has at least one peak in the region from 1705 to 1740 cm-1 and at least two peaks, for example at least three, at least four, at least five, at least six, at least seven, at least eight, at least nine or at least ten peaks in the region from 1400 to 900 cm-1 in a ATR_FTIR. In particular, the oxyfunctionalized polyolefin plastic has at least one peak in the region from 1724 to 1740 cm-1 and at least two peaks, for example at least three, at least four, at least five, at least six, at least seven, at least eight, at least nine or at least ten peaks in the region from 1400 to 900 cm-1 in a ATR_FTIR. For example, the oxyfunctionalized polyolefin comprises the following peaks in a ATR_FTIR spectrum: 1740 +/- 4 cm-1, 1724 +/- 4 cm-1, 1263.21 +/- 4 cm-1, 1229.50 +/- 4 cm-1, 1184.91 +/- 4 cm-1, 1133.02+/- 4 cm-1, 1100.82 +/- 4 cm-1, 1058.07 +/- 4 cm-1, 980.70 +/- 4 cm-1, and 894.71 +/- 4 cm-1. In particular, the oxyfunctionalized polyolefin is oxyfunctionalized polyethylene or polypropylene, particularly polyethylene, and it has the peaks in the ATR_FTIR spectrum as described above.

A fourth aspect of the invention provides for the use of an isolated short UPO in polyolefin oxyfunctionalization, which results in reducing the polyolefin hydrophobicity, in polyolefin degradation, in polyolefin depolymerization, wherein the use is in aqueous media and in the presence of an hydroperoxide. In one embodiment of the fourth aspect, the short UPO is for use in combination with another enzyme. In a particular embodiment, the other enzyme may be a ligninolytic oxidoreductase enzyme different from short UPO, such as a long UPO, a laccase, a manganese peroxidase and a versatile peroxidase.

Throughout the description and claims the word "comprise" and variations of the word, are not intended to exclude other technical features, additives, components, or steps. Furthermore, the word "comprise" encompasses the case of "consisting of". Additional advantages and features of the invention will become apparent to those skilled in the art upon examination of the description or may be learned by practice of the invention. The following examples and drawings are provided by way of illustration, and they are not intended to be limiting of the present invention.

### Examples

### MATERIALS and METHODOLOGY

### Reagents and enzymes

Low density Polyethylene (LDPE) pellets were produced by Repsol S.A. ABTS (2,2'-azino-bis(3-ethylbenzothiazolin-6-sulfonic) acid), DMP (2,6-dimetoxiphenol), and the Saccharomyces cerevisiae transformation kit were obtained from Sigma-Aldrich (Saint Louis, MO, USO). NBD (5-nitro-1,3-benzodioxole) was acquired from TCI America (Portland, OR, USA). The mut⁺ strain X-33 strain was obtained from ThermoFisher ((ThermoFisherScientific, US). The construction for UPO3 of *M. rotula* expression, including cDNA, signal peptide, and vector was performed as described in Püllmann et al. 2021.

### Cloning, expression and purification of enzymes.

Cloning, expression and purification of short UPO from *Marasmius rotula* (UPO3) were conducted according to the procedures described by Püllmann et al. 2021.

Cloning, expression and purification of long UPO from *Agrocybe aegerita* (UPO1) were conducted according to the procedures described by Gomez de Santos et al. 2018.

Cloning, expression and purification of laccase were conducted according to the procedures described by Gomez-Fernández et al. 2020.

Cloning, expression and purification of versatile peroxidase were conducted according to the procedures described by Gonzalez-Perez et al. 2016.

### Determination of the natural activity of enzymes

The residual activity of the enzymes was controlled through the measurement of their natural activity using colorimetric assays.

For the detection of UPO3 activity, the 2,6-dimethoxyphenol (DMP) reaction mixture was composed of 100 mM pH 5.0 citrate phosphate buffer, 2 mM DMP, and 5 mM H₂O₂. The mix was briefly stirred and absorbance was measured at 469 nm following it in kinetic mode using a plate reader for such purpose (SPECTRAMax Plus 384, Molecular Devices, USA).

For the detection of UPO1 activity, the ABTS reaction mixture was composed of 100 mM pH 4.0 citrate phosphate buffer, 0.2 mM ABTS and 2 mM H₂O₂. The mix was briefly stirred and absorbance was measured at 418 nm following it in kinetic mode using a plate reader for such purpose (SPECTRAMax Plus 384, Molecular Devices, USA).

For the detection of laccase activity, the ABTS reaction mixture was composed of 100 mM pH 4.0 citrate phosphate buffer and 1 mM ABTS. The mix was briefly stirred and absorbance was measured at 418 nm following it in kinetic mode using a plate reader for such purpose (SPECTRAMax Plus 384, Molecular Devices, USA).

For the detection of versatile peroxidase activity, the ABTS reaction mixture was composed of 100 mM pH 4.0 citrate phosphate buffer, 2 mM ABTS and 0.1 mM H₂O₂. The mix was briefly stirred and absorbance was measured at 418 nm following it in kinetic mode using a plate reader for such purpose (SPECTRAMax Plus 384, Molecular Devices, USA).

### Thermo-oxidation of LDPE (pellet)

Ground pellets of LDPE were placed in an oven at 96°C for three weeks. Pellets were daily mixed with a scraper and the level of oxidation was followed through weekly analysis of the LDPE using ATR- FT-IR.

### Enzymatic modification of LDPE

13 mg of LDPE pellets and 3 µM of pure UPO3 in 100 mM pH 5.0 citrate phosphate buffer and 5 mM H₂O₂ were charged in a 5 mL glass vial. The vials were placed in a roller (Movil-Rod, JPSELECTA, Spain) inside of a temperature chamber with a set temperature of 30°C. To keep the supply of H₂O₂ an aliquot of 25 µl at 200 mM of H₂O₂ was added 3 times per day. Every 48 hours the supernatant was extracted from the vials and replaced with a freshly new prepared reaction mixture. Reactions were kept for two weeks or four (see Results).

The reaction conditions with UPO1 were the same as for UPO3 except for the reaction mixture which contained 3 µM of pure UPO1 in 100 mM pH 7.0 potassium phosphate buffer and 2 mM H2O2, instead.

The reaction conditions with laccase were the same as for UPO3 except for the reaction mixture which contained 0.05 µM of pure laccase in 100 mM pH 4.5 citrate phosphate buffer and 1 mM of HBT, instead.

The reaction conditions with versatile peroxidase were the same as for UPO3 except for the reaction mixture which contained 0.05 µM of pure versatile peroxidase in 100 mM pH 5.0 malonate buffer, 0.1 mM of H₂O₂, and 1.2 mM of Mn²⁺, instead.

Negative controls of each enzyme reaction were prepared and the conditions and composition of their mixtures were the same as the ones described above but adding water instead of the enzyme.

### Cleaning of LDPE samples

LDPE samples were collected into 1.5 mL microtubes with nylon filter (0.45 µm) (CORNING, USA). Samples were centrifuged at 12,000 rpm (RT) for 2 min, and the reaction mixture was discarded. SDS at 2% was added, tubes vortexed and centrifuged at 12,000 rpm (RT) for 2 min. The SDS was removed and new SDS 2% was added, tubes vortexed and then, incubated for 1 hour in a shaker at 30°C, 220 RPM. SDS was discarded and dH₂O water was added to the tubes. Samples were vortexed and centrifuged as aforementioned, two times. Then, cap-opened tubes were incubated in an oven at 60°C for 1 hour to get them dry.

### Infrared analysis of LDPE

Changes in LDPE structure following incubation with enzymes were analyzed by ATR FT-IR (GladiATR, PIKE Technologies, USA). After establishing a blank, the FT-IR performs 128 scanners between 4000-600 cm⁻¹ of the sample. Enzymatic oxidation of the LDPE was measured as the carbonyl index (CI), defined as the ratio of the maximum absorbance in the carbonyl area to the reference bands of CH₂ and CH₃ which served as internal standard (*i.e* the ratio of the absorbance peak of carbonyl at region 1800-1700 cm⁻¹ and CH₂, CH₃ stretching bands at 3000-2750 cm ⁻¹).

### RESULTS

### Oxidation of LDPE with unspecific peroxygenase vs other enzymes

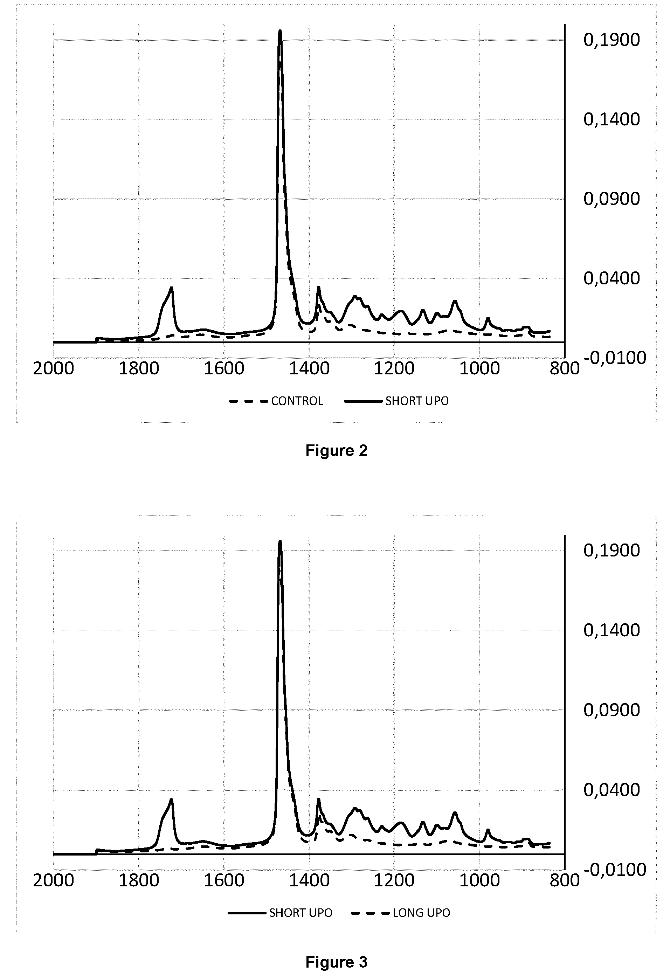

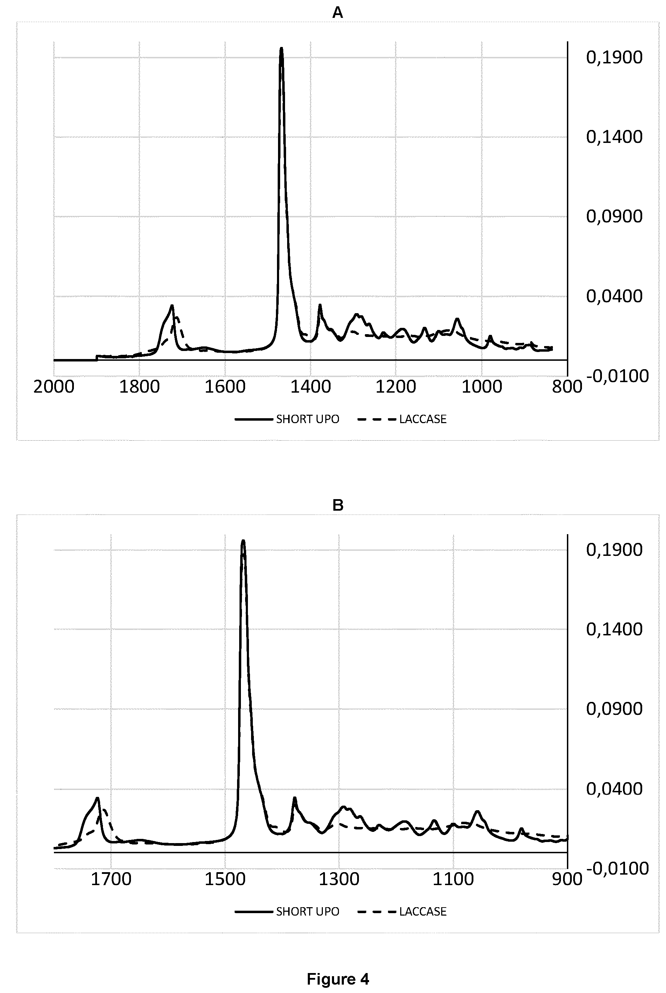

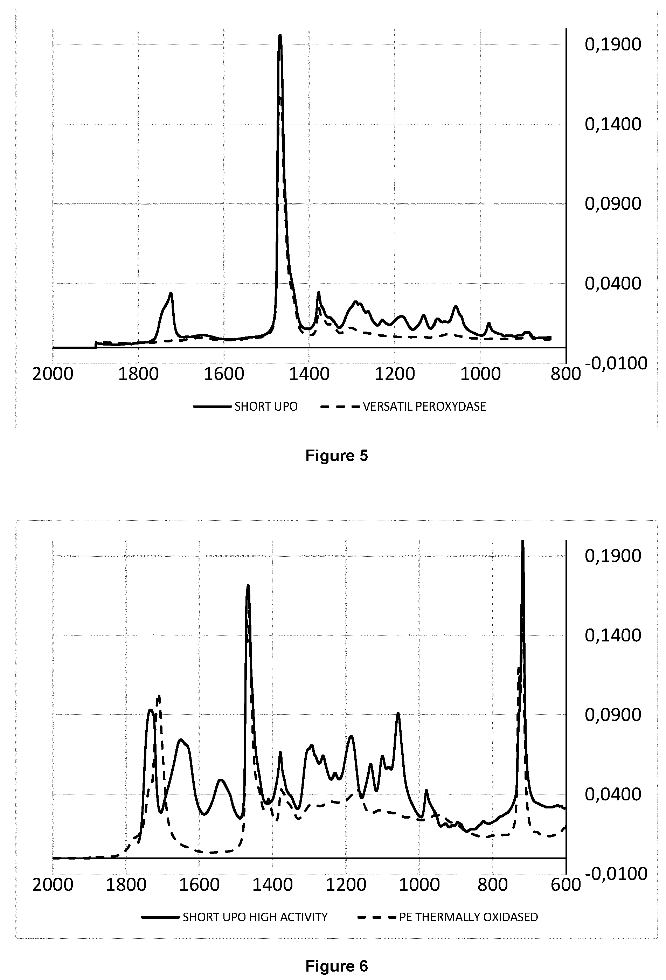

Pure extracts of UPO3, UPO1, laccase, and versatile peroxidase were mixed with LDPE in the conditions described in the Materials and Methods section. After two weeks of treatment, the LDPE samples were isolated from the reaction mix and cleaned. The analysis by FT-IR of the LDPE treated with UPO3 and laccase showed an increment of the CI in comparison with negative control (0.0313 and 0.0294, respectively), Figure 2, 4. On the contrary, UPO1 and versatile peroxidase did not render any measurable changes in the material surface, Figure 3, 5.

The modifications in the surface of the LDPE are different if the process is performed with UPO3 or laccase. With laccase, only a clear peak at 1712 cm⁻¹ is generated. When the employed enzyme is the UPO3, the clearest signal corresponds to the peak at 1724 cm⁻¹ but the shape of the peak makes evident that there is more than one carbonyl group. Furthermore, the region between 1400-900 cm⁻¹ is highly altered (1281,77 cm⁻¹ 1263,21 cm⁻¹ 1229,50 cm⁻¹ 1184,91 cm⁻¹ 1133,02 cm⁻¹ 1100,82 cm⁻¹ 1058,07 cm⁻¹ 980,70 cm⁻¹ and 894,71 cm⁻¹) in comparison with the negative control or laccase treated LDPE, Figure 4.

### Oxidation of LDPE with unspecific peroxygenase vs other techniques

A pure extract of UPO3 was mixed with LDPE in the conditions described in the Materials and Methods section for four weeks. On the other side, LDPE samples were submitted to a process of thermo-oxidation as described in the Materials and Methods section. The LDPE samples were isolated, cleaned, and analyzed by FT-IR. The CI calculated for the LDPE treated with the enzyme was 0.113, while the "thermo-oxidized" of the LDPE reached a value of 0.162. The thermo-oxidized material has no other changes in the IR compared with the native material. Conversely, the material treated with the UPO3 exhibits new signals in the region between 1400-900 cm⁻¹ no present in the thermo-oxidized material, Figure 6.

### Citation List

Altschul et al., "Basic local alignment search tool", 1990, J. Mol. Biol, vol. 215, pp. 403-410.
EMBOSS: The European Molecular Biology Open Software Suite, Rice et al., 2000, Trends Genet. 16: 276-277
Gomez de Santos, P., Cañellas, M., Tieves, F., Younes, S. H., Molina-Espeja, P.,
Hofrichter, M., Hollmann, F., Guallar, V., Alcalde, M. 2018. Selective synthesis of the human drug metabolite 5' -hydroxypropranolol by an evolved self-sufficient peroxygenase. ACS Catalysis, 8(6), 4789-4799.
Gomez-Fernandez, B. J., Risso, V. A., Sanchez-Ruiz, J. M., Alcalde, M. 2020. Consensus design of an evolved high-redox potential laccase. Frontiers in Bioengineering and Biotechnology, 8, 354.
Gonzalez-Perez, D., Mateljak I., Garcia-Ruiz, E., Ruiz-Dueñas F. J., Martinez, A. T., Alcalde, M. 2016. Alkaline versatile peroxidase by directed evolution. Catal. Sci. Technol. 6, 6625-6636.
Hobisch, M., van Schie, M.M.C.H., Kim, J., Andersen, K.R., Alcalde, M., Kourist, R., Park, C.B., Hollmann, F., Kara, S., 2020. Solvent-Free Photobiocatalytic Hydroxylation of Cyclohexane. ChemCatChem 12, 40009.
Hofrichter, M., Kellner, H., Pecyna, M.J., Ullrich, R., 2015. Fungal Unspecific Peroxygenases: Heme-Thiolate Proteins That Combine Peroxidase and Cytochrome P450 Properties. Adv Exp Med Biol, 2015;851:341-68. doi: 10.1007/978-3-319-16009-2_13.
Needleman and Wunsch, 1970, J. Mol. Biol. 48, 443-453.
Püllmann P, Knorrscheidt A, Münch J, Palme PU, Hoehenwarter W, Marillonnet S, Alcalde M, Westermann B, Weissenborn MJ. 2021. A modular two yeast species secretion system for the production and preparative application of unspecific peroxygenases. Commun Biol 4, 562.
Ullrich, R., Poraj-Kobielska, M., Scholze, S., Halbout, C., Sandvoss, M., Pecyna, M.J., Scheibner, K., Hofrichter, M., 2018. Side chain removal from corticosteroids by unspecific peroxygenase. J. Inorg. Biochem. 183, 84-93.
van Schie, M.M.C.H., Kaczmarek, A.T., Tieves, F., Gomez de Santos, P., Paul, C.E., Arends, I.W.C.E., Alcalde, M., Schwarz, G., Hollmann, F., 2020. Selective Oxyfunctionalisation Reactions Driven by Sulfite Oxidase-Catalysed In Situ Generation of H2O2. ChemCatChem 12, 3186-3189.
van Schie, M.M.C.H., Zhang, W., Tieves, F., Choi, D.S., Park, C.B., Burek, B.O., Bloh, J.Z., Arends, I.W.C.E., Paul, C.E., Alcalde, M., Hollmann, F., 2019. Cascading g-C3N4 and Peroxygenases for Selective Oxyfunctionalization Reactions. ACS Catal. 9, 7409-7417.
Sakhalkar S. et al., "Screening and Identification of Soil Fungi with Potential of Plastic Degrading Ability", Indian Journal of Applied Research, 31 December 2013, pages 62-64.
Scheibner K. et al., "Unspezifische Peroxygenasen - Oxyfunktionalisierung außerhalb der Pilzhyphe", Biospektrum, vol. 26, no. 1, 1 February 2020, pages 103-106.
US 2020/40632 A1.
WO 2008/012236 A1.

## Claims

1. A method for oxyfunctionalization of polyolefin, said method comprising the step of (i) contacting the polyolefin with an isolated short unspecific peroxygenase enzyme (short UPO) in aqueous media and in the presence of an hydroperoxide, wherein the concentration of hydroperoxide ranges from 0.1 to 10 mM, in particular from 1 to 10 mM.

2. The method according to claim 1, wherein the short UPO has a sequence identity of at least 70% with SEQ ID NO: 1, or has a sequence identity of at least 90% with SEQ ID NO: 1, or has a sequence identity of at least 98% with SEQ ID NO: 1.

3. The method according to any one of the preceding claims, wherein the pH at which step (i) takes place ranges from 3 to 8, in particular from 4 to 6.

4. The method according to any one of the preceding claims, wherein the hydroperoxide is selected from the group consisting of H₂O₂, t-butyl hydroperoxide, and cumene hydroperoxide.

5. The method according to any one of the preceding claims, wherein the amount of short UPO in the contacting step ranges from 0.1 to 100 µM.

6. The method according to any one of the preceding claims, wherein the media comprises citrate.

7. The method according to any one of the preceding claims, wherein the temperature at which step (i) takes place ranges from 10 to 70 °C, in particular, from 20 to 50 °C.

8. The method according to any one of the preceding claims, said method further comprising a step (ii) of contacting the plastic with another Ligninolytic Oxidoreductase enzyme different from short UPO.

9. The method according to any one of the preceding claims, wherein the polyolefin is polyethylene.

10. A method for degrading polyolefin, said method comprising oxyfunctionalization of the plastic as defined in any one of the preceding claims.

11. The method for degrading polyolefin according to claim 10, said method further comprising disposal of the oxyfunctionalized plastic, for example, in a landfill.

12. A method for depolymerizing a polyolefin, said method comprising oxyfunctionalization of the polyolefin by a method as defined in any one of claims 1-9.

13. Use of an isolated short UPO in polyolefin oxyfunctionalization, in polyolefin degradation, or in polyolefin depolymerization, wherein the use is in aqueous media and in the presence of an hydroperoxide.

## Patentansprüche

1. Ein Verfahren zur Oxyfunktionalisierung von Polyolefin, wobei das Verfahren den folgenden Schritt umfasst: (i) In-Kontakt-bringen des Polyolefins mit einem isolierten kurzen unspezifischen Peroxygenase-Enzym (kurzen UPO, *unspecific peroxygenase*) in einem wässrigen Medium und in Gegenwart eines Hydroperoxids, wobei die Konzentration des Hydroperoxids im Bereich von 0,1 bis 10 mM, insbesondere von 1 bis 10 mM, liegt.

2. Das Verfahren nach Anspruch 1, wobei die kurze UPO eine Sequenzidentität von mindestens 70% mit der SEQ ID NO: 1, oder eine Sequenzidentität von mindestens 90 % mit der SEQ ID NO: 1, oder eine Sequenzidentität von mindestens 98 % mit der SEQ ID NO: 1 hat.

3. Das Verfahren nach einem der vorhergehenden Ansprüche, wobei der pH-Wert, bei dem der Schritt (i) stattfindet, von 3 bis 8, insbesondere von 4 bis 6, reicht.

4. Das Verfahren nach einem der vorhergehenden Ansprüche, wobei das Hydroperoxid ausgewählt ist aus der Gruppe bestehend aus H₂O₂, t-Butylhydroperoxid und Cumolhydroperoxid.

5. Das Verfahren nach einem der vorhergehenden Ansprüche, wobei die Menge an kurzer UPO in dem Kontaktierungsschritt von 0,1 bis 100 µM reicht.

6. Das Verfahren nach einem der vorhergehenden Ansprüche, wobei das Medium Citrat umfasst.

7. Das Verfahren nach einem der vorhergehenden Ansprüche, wobei die Temperatur, bei der der Schritt (i) stattfindet, von 10 bis 70 °C, insbesondere von 20 bis 50 °C, reicht.

8. Das Verfahren nach einem der vorhergehenden Ansprüche, wobei das Verfahren ferner einen Schritt (ii) des In-Kontakt-bringens des Kunststoffs mit einem anderen ligninolytischen Oxidoreduktase-Enzym, das sich von der kurzen UPO unterscheidet, umfasst.

9. Das Verfahren nach einem der vorhergehenden Ansprüche, wobei das Polyolefin Polyethylen ist.

10. Ein Verfahren zum Abbau von Polyolefin, wobei das Verfahren die Oxyfunktionalisierung des Kunststoffs, wie in einem der vorhergehenden Ansprüche definiert, umfasst.

11. Das Verfahren zum Abbau von Polyolefin nach Anspruch 10, wobei das Verfahren ferner die Entsorgung des oxyfunktionalisierten Kunststoffs, zum Beispiel in einer Deponie, umfasst.

12. Ein Verfahren zum Depolymerisieren eines Polyolefins, wobei das Verfahren die Oxyfunktionalisierung des Polyolefins durch ein Verfahren wie in einem der Ansprüche 1 bis 9 definiert umfasst.

13. Verwendung einer isolierten kurzen UPO bei der Polyolefin-Oxyfunktionalisierung, beim Polyolefin-Abbau oder bei der Polyolefin-Depolymerisation, wobei die Verwendung in einem wässrigen Medium und in Gegenwart eines Hydroperoxids erfolgt.

## Revendications

1. Un procédé pour oxyfonctionnaliser une polyoléfine, ledit procédé comprenant l'étape de (i) mettre en contact la polyoléfine avec une enzyme peroxygénase non spécifique courte (UPO, *unspecific peroxygenase enzyme,* courte) isolée dans un milieu aqueux et en présence d'un hydroperoxyde, dans lequel la concentration d'hydroperoxyde va de 0,1 à 10 mM, en particulier de 1 à 10 mM.

2. Le procédé selon la revendication 1, dans lequel l'UPO courte a une identité de séquence d'au moins 70 % avec la SEQ ID n ° : 1, ou a une identité de séquence d'au moins 90 % avec la SEQ ID n ° : 1, ou a une identité de séquence d'au moins 98 % avec la SEQ ID n ° : 1.

3. Le procédé selon l'une quelconque des revendications précédentes, dans lequel le pH auquel l'étape (i) a lieu va de 3 à 8, en particulier de 4 à 6.

4. Le procédé selon l'une quelconque des revendications précédentes, dans lequel l'hydroperoxyde est choisi dans le groupe constitué par H₂O₂, l'hydroperoxyde de t-butyle et l'hydroperoxyde de cumène.

5. Le procédé selon l'une quelconque des revendications précédentes, dans lequel la quantité d'UPO courte dans l'étape de mise en contact va de 0,1 à 100 µM.

6. Le procédé selon l'une quelconque des revendications précédentes, dans lequel le milieu comprend du citrate.

7. Le procédé selon l'une quelconque des revendications précédentes, dans lequel la température à laquelle l'étape (i) a lieu va de 10 à 70 °C, en particulier de 20 à 50 °C.

8. Le procédé selon l'une quelconque des revendications précédentes, ledit procédé comprenant en outre une étape (ii) de mise en contact du plastique avec une autre enzyme ligninolytique oxydoréductase différente de l'UPO courte.

9. Le procédé selon l'une quelconque des revendications précédentes, dans lequel la polyoléfine est le polyéthylène.

10. Un procédé pour dégrader une polyoléfine, ledit procédé comprenant l'oxyfonctionnalisation du plastique tel que définie dans l'une quelconque des revendications précédentes.

11. Le procédé pour dégrader une polyoléfine selon la revendication 10, ledit procédé comprenant en outre l'élimination du plastique oxyfonctionnalisé, par exemple, dans une décharge.

12. Un procédé pour dépolymériser une polyoléfine, ledit procédé comprenant l'oxyfonctionnalisation de la polyoléfine par un procédé tel que défini dans l'une quelconque des revendications 1 à 9.

13. Utilisation d'une UPO courte isolée dans l'oxyfonctionnalisation de polyoléfine, dans la dégradation de polyoléfine, ou dans la dépolymérisation de polyoléfine, dans laquelle l'utilisation est en milieu aqueux et en présence d'un hydroperoxyde.
